# EUROPÄISCHE PATENTSCHRIFT

(11) **EP 1 455 806 B1**
(45) Veröffentlichungstag und Bekanntmachung des Hinweises auf die Patenterteilung: **08.07.2009**
(21) Anmeldenummer: 02798286.7
(22) Anmeldetag: 19.12.2002
(51) Int. Cl.: A61Q 7/00, A61K 8/97, A61K 36/39

(54) **WIRKSTOFFMISCHUNG ENTHALTEND EIN PFLANZENEXTRAKT VON CONVOLVULACEAE ZUR BEHANDLUNG VON HAARPROBLEMEN, HAUTSTÖRUNGEN, HAUTKRANKHEITEN**
ACTIVE AGENT MIXTURE CONTAINING A PLANT EXTRACT OF CONVOLVULACEAE FOR TREATING HAIR PROBLEMS, SKIN DISORDERS AND SKIN DISEASES
MELANGE DE PRINCIPES ACTIFS CONTENANT UN EXTRAIT VEGETAL DE CONVOLVULACEAE, UTILISE POUR TRAITER LES PROBLEMES CAPILLAIRES, LES PROBLEMES CUTANES ET LES MALADIES DE LA PEAU

(30) Priorität: 21.12.2001 DE 10163190
(43) Veröffentlichungstag der Anmeldung: 15.09.2004
(73) Patentinhaber: Kalander Barzinjy, 64297 Darmstadt (DE)
(72) Erfinder: SHAHO, Ismail Barzanji, Hawler, Kurdistan (IQ)
(86) Internationale Anmeldenummer: PCT/DE2002/004670
(87) Internationale Veröffentlichungsnummer: WO 2003/055503

(56) Entgegenhaltungen:
- WO-A-01/91791
- WO-A-01/97825
- DE-A- 19 927 229
- HIBIBINYA M ET AL: "PRELIMINARY REPORT ON ANTITUMOR ACTIVITY OF IRANIAN MEDICINAL PLANTS" PLANTA MEDICA, THIEME, STUTTGART, DE, Bd. 59, Nr. 7, 1993, Seiten A682-A683, XP000914722 ISSN: 0032-0943

## Beschreibung

Die Erfindung betrifft eine Wirkstoffmischung zur Herstellung eines Mittels für die therapeutische und/oder kosmetische Behandlung von Haarproblemen und/oder anderen Krankheiten und Störungen der Haut.

Probleme mit dem Haar - sei es Haarausfall, greises Haar oder kraftloses, dünnes Haar. Sie belasten viele der Betroffenen stark und schränken damit deren Lebensqualität erheblich ein.

Hautkrankheiten, die mit Juckreiz, Pigmentstörungen oder andere Symptome einhergehen, haben einen noch viel negativeren Einfluß auf die Lebensqualität der Betroffenen.

Der Stand der Technik umfaßt eine Reihe von Mitteln, die zur Bekämpfung einzelner der genannten Phänomene vorgesehen sind.

Bekannt sind verschiedene Haarwuchsmittel - wenige davon auf schonender und umweltfreundlicher pflanzlicher Basis - deren Wirksamkeit jedoch höchst unterschiedlich und generell wenig zufriedenstellend ist. Viele wirken nur spezifisch bei bestimmten Ursachen für den Haarausfall, nur wenige wirken breitbandiger bei mehreren Ursachen. Den meisten Mitteln gemeinsam sind mehr oder weniger starke Nebenwirkungen.

Gegen graues Haar ist ein Mittel bekannt, das dem ergrautem Haar seine ursprüngliche Farbe zurückgibt. Diese Mittel bewirkt jedoch nicht, daß das Haar in der Originalfarbe weiter wächst. Es verhindert auch nicht das Ergrauen weiterer Haare.

Gegen Hautkrankheiten gibt es zahllose, spezifisch wirksame Medikamente. Pilzinfektionen, Pigmentstörungen, Juckreiz und Schuppenflechte können mit diesen Mitteln mehr oder weniger erfolgreich behandelt werden, wobei jeweils mehr oder weniger starke Nebenwirkungen in Kauf genommen werden müssen. Bisher gibt es noch kein Mittel, das lindernd oder heilend auf alle die genannten Krankheiten wirkt.

Aufgabe der vorliegenden Erfindung ist die Bereitstellung eines Mittels, das positiv bis hin zur vollständigen Heilung und breitbandig auf die vorstehend genannten Haar- und Hautprobleme bzw. -krankheiten einwirkt und dabei frei oder annähernd frei von Nebenwirkungen ist.

Eine Lösung dieser Aufgabe besteht in der Bereitstellung einer Wirkstoffmischung der eingangs genannten Art, die dadurch gekennzeichnet ist, daß die Wirkstoffmischung aus Pflanzen der Familie Convolvulaceae gewonnen wird, indem aus den Pflanzen entweder (i) ein wässriger Sud durch Kochen in Wasser oder (ii) ein alkoholischer Auszug durch Einlegen in Alkohol oder (iii) eine Paste durch Verarbeitung der fein zerriebenen Pflanze mit hautverträglichen mehr oder minder viskosen Flüssigkeiten hergestellt wird.

Im Rahmen der Untersuchungen, die zu der vorliegenden Erfindung geführt haben, wurde nämlich überraschenderweise gefunden, daß diese pflanzliche Wirkstoffmischung - im folgenden Shaho-Präparat genannt - die Haut so weit belebt und stärkt, daß nachweislich folgende Wirkungen erzielt werden:
■ Stoppen von Haarausfall
■ Anregung von neuem Haarwachstum
■ Vitalisierung vorhandener Haarwurzeln
■ Stabilisierung und Wiederbelebung der Haarpigmentproduktion
■ Normalisierung der Hautpigmentproduktion
■ Vitalisierung trockener, dünner oder auf andere Arten degenerierter Haut
■ Verbesserung der Symptome von Schuppenflechte bis hin zur Heilung

Das Shaho-Präparat wirkt auf eine Vielzahl von Krankheiten und Störungen der Haut. Es ist einfach und umweltfreundlich herzustellen, ist gut verträglich und hat praktisch keine Nebenwirkungen.
In Kombination mit anderen einfachen pflanzlichen Wirkstoffen kann die Wirkung auf bestimmte Erkrankungen gezielt verstärkt werden (wie im weiteren beschrieben wird). Die gute Verträglichkeit erlaubt einen vorbeugenden Einsatz sowie die Nutzung für kosmetische Zwecke.

Die Wirkstoffmischung des Shaho-Präparates wird vorzugsweise aus Pflanzen der Gattung *Convolvulus euphraticus,* insbesondere der Art *Convolvulus euphraticus Bornm.* gewonnen.

Gegenstand der Erfindung ist deshalb auch eine Wirkstoffmischung an sich, die dadurch gekennzeichnet ist, daß sie aus einer Pflanze der Art *Convolvulus euphraticus Bornm.* gewonnen wird, indem aus der Pflanze entweder (i) ein wässriger Sud durch Kochen in Wasser oder (ii) ein alkoholischer Auszug durch Einlegen in Alkohol oder (iii) eine Paste durch Verarbeitung der fein zerriebenen Pflanze mit hautverträglichen mehr oder minder viskosen Flüssigkeiten gewonnen wird, wobei der Sud bzw. der Auszug bzw. die Paste die Wirkstoffmischung enthält.

In der praktischen Anwendung hat sich gezeigt, daß die erfindungsgemäße Wirkstoffmischung insbesondere zur Herstellung eines Mittel gegen Haarausfall (eines Haarwuchsmittels) geeignet ist, wobei dieses Mittel eine sehr gute Wirksamkeit zeigt.

Die erfindungsgemäße Wirkstoffmischung ist auch zur Herstellung eines Mittels für die Vitalisierung und Kräftigung vorhandener Haarwurzeln und damit für den Erhalt von gesünderem, kräftigerem und schönerem Haar mit gutem Erfolg einsetzbar.

Desweiteren ist die erfindungsgemäße Wirkstoffmischung zur Herstellung eines Mittels geeignet, welches die Hautpigmentproduktion anregt und damit die Zunahme grauer Haare stoppt und ergraute Haare in ihrer ursprünglichen Farbe weiter wachsen läßt.

Die erfindungsgemäße Wirkstoffmischung eignet sich auch zur Herstellung eines Mittels, welches die Hautpigmentproduktion normalisiert und damit sowohl zu hellen als auch zu dunklen Bereichen der Haut ihre ursprüngliche Farbe wiedergibt.

Ein weiterer Einsatz der erfindungsgemäßen Wirkstoffmischung besteht in der Herstellung eines Mittels, welches zur Vitalisierung trockener, dünner oder auf andere Arten degenerierter Haut geeignet ist.

Die erfindungsgemäße Wirkstoffmischung ist nicht zuletzt insbesondere auch für die Herstellung eines Mittels zur Heilung von Schuppenflechte (Psoriasis), eines Mittels zur Heilung von Infektionskrankheiten der Haut, eines Mittels zur Linderung von Hautjucken sowie eines Mittels zur Linderung von Neurodermitis vorgesehen.

Mit der erfindungsgemäßen Wirkstoffmischung gehen insbesondere die folgenden Vorteile einher:
- Haarausfall wurde bei fast allen Testpersonen (>90%) gestoppt, unabhängig von der Ursache. Sowohl erblich bedingter Haarausfall des Mannes (*androgenic alopecia*) als auch andere Formen des Haarausfalls bei Mann und Frau konnten aufgehalten werden (u.a. auch *alopecia areate,* der sog. Kreisrunde Haarausfall).
   Bestes Ergebnis war ein vollkommener Stopp des Haarausfalls nach einer Woche, schlechtestes Ergebnis war nach 3 Wochen eine Reduktion des Ausfalls um ein Drittel.
   Bei Haarausfall aufgrund *androgenic alopecia* trat der Erfolg meist innerhalb von 10 Tagen ein. Haarausfall durch *alopecia areate* wurde innerhalb von 4 bis 8 Wochen bei allen betroffenen Testpersonen gestoppt.
- Neues Haarwachstum konnte bei über 90% der Testpersonen beobachtet werden. Dichte, Wachstumsgeschwindigkeit und notwendige Behandlungsdauer waren abhängig davon, wie lange die behandelten Stellen schon kahl waren.
   Selbst auf seit Jahren kahlen Stellen hat sich in fast allen Fällen nach 3 Monaten ein flächendeckender dünner Flaum gebildet, zusätzlich zu einzelnen 4 bis 7 cm langen Haaren. In einem Einzelfall kam es nach zwischenzeitlichem erneuten Haarausfall 6 Monate nach Abbruch der Behandlung zu flächendeckendem dichten Wachstum neuer kräftiger gesunder Haare.
   In weniger schweren Fällen war der Erfolg entsprechend größer und auch schneller zu erzielen.
- Glanz- und kraftlose dünne Haare sind nach ein- bis dreiwöchiger Behandlung in allen Fällen gesünder, kräftiger und schöner weiter gewachsen. Diese Wirkung blieb nach Ende der Behandlung langfristig erhalten.
- Haare, die erst seit 3 Monaten oder weniger ergraut waren, wuchsen nach ca. zehntägiger Behandlung wieder mit der alten Farbe.
   Die Ergrauung weiterer Haare wurde aufgehalten bzw. verlangsamt. Etwa 90% der Haare behielten im Schnitt dauerhaft ihre Originalfarbe bei.
- Hautpigmentstörungen, die erst seit wenigen Monaten aufgetreten waren, konnten nach vier- bis sechsmonatiger Behandlung geheilt werden, sowohl helle als auch dunkle Hautflecken.
   Die Behandlung zeigte bei allen Testpersonen eine generell belebende Wirkung auf die Haut.
   Dünne Hautstellen, Haut mit Störungen von Schweiß- und Talgdrüsen, Haut mit degeneriertem Binde- und Fettgewebe verbesserte ihren Zustand mit der Dauer der
   Behandlung. Spätestens nach 2 bis 4 Monaten Behandlung hatten solche Hautstellen die Fähigkeit wiedererlangt, Schweiß und Talg in genügender Menge zu produzieren.
   Das Bindegewebe hatte bei den meisten Testpersonen seine alte Elastizität wiedererhalten, und das nach etwa 3 Wochen.
- Die Behandlung von Schuppenflechte war bei allen Betroffenen erfolgreich Nach spätestens 8 Wochen waren die Symptome verschwunden
- Pilzinfektionen der Haut waren nach etwa 3-4 Wochen bei allen behandelten Testpersonen abgeheilt.
   Eine positive Wirkung bei anderen Infektionskrankheiten der Haut ist sehr wahrscheinlich.
- Hautjucken aufgrund von Allergien oder z.B. aufgrund extrem fettiger Kopfhaut ist bei den behandelten Personen nach etwa 4 Wochen verschwunden.

Die Erfindung wird im folgenden anhand von Ausführungsbeispielen näher erläutert.

### Beispiel 1: Gewinnung eines alkoholfreien Shaho-Präparats

Von wildwachsenden oder in Kultur wachsenden Pflanzen der Familie *Convolvulaceae,* vorzugsweise der Gattung *Convolvulus euphraticus* und besonders bevorzugt der Art *Convolvulus euphraticus Bornm.,* werden alle oberirdischen Pflanzenteile geerntet. Bevorzugte Erntezeit ist Juli bis September.
Die Pflanzenteile werden in frisch geernteter oder in getrockneter Form verwendet. Vor der Verarbeitung sind sie gut zu waschen.
Die Pflanzenteile werden 6 Stunden lang in siedendem Wasser gekocht, wobei auf 1 Volumenteil locker geschichtetes Pflanzenmaterial 2,5 Volumenteile Wasser gegeben werden.
Während des Kochvorgangs zerfallen die Pflanzenteile nicht, sondern sie saugen einen großen Teil der Flüssigkeit auf, die restliche Flüssigkeit ist grün gefärbt.
In einer Presse wird mit einem Druck von 5 bis 10 bar wird die Flüssigkeit vom festen Pflanzenmaterial getrennt.
Die Flüssigkeit, d.h. der Sud, wird weiterverarbeitet, nämlich eingedickt, bis er etwa die Viskosität von Zahnpasta (oder ein wenig flüssiger) erreicht hat.
Das Eindicken kann durch Einkochen oder durch Verdunsten bei Raumtemperatur, beispielsweise im Sonnenlicht, erfolgen.
In Probandenversuchen hat sich gezeigt, daß durch Verdunstung im Sonnenlicht eingedickter Sud eine noch höhere Wirksamkeit erzielt als eingekochter Sud.

### Beispiel 2: Gewinnung eines Shaho-Präparats mittels alkoholischer Extraktion

Pflanzenteile werden wie in Beispiel 1 beschrieben geerntet und gewaschen bzw. geputzt. Anschließend werden die Pflanzenteile kleingeschnitten, im Volumenverhältnis 1:1 (locker geschichtetes Pflanzenmaterial zu Alkohol) in 70- bis 95vol%igen Alkohol (% Alkohol in Wasser) eingelegt und 15-20 Tage lang (oder länger) in einem luftdicht verschlossenen Gefäß aufbewahrt.
Die Pflanze zerfällt dabei nicht, sondern saugt einen großen Teil der Flüssigkeit auf. Die restliche Flüssigkeit ist braun gefärbt.
In einer Presse wird mit einem Druck von ca. 5 bar die Flüssigkeit vom festen Pflanzenmaterial getrennt. Weiterverarbeitet wird die Flüssigkeit, d.h. der Sud.
Der Sud wird entweder direkt als flüssiges Shaho-Präparat (Tinktur) eingesetzt (siehe Beispiel 4, "Shaho-F") oder durch Sonnen- und/oder Lufttrocknung so weit eingedickt, bis er etwa die Viskosität von Zahnpasta oder ein wenig flüssiger erreicht hat.

### Beispiel 3: Gewinnung eines Shaho-Präparats durch Zerreiben

Pflanzenteile werden wie in Beispiel 1 beschrieben geerntet und gewaschen bzw. geputzt. Anschließend werden die Pflanzenteile mechanisch zerkleinert und zu einer Paste verriebenen.

### Beispiel 4: Gewinnung von Shaho-Cremes (Shaho-Salben) und Shaho-Flüssigpräparaten für die Anwendung in Probandentests

### Shaho-A (Creme/Salbe)

100 Gewichtsteile des gemäß Beispiel 1 oder Beispiel 2 hergestellten eingedickten Suds werden mit 100 Gewichtsteilen eines natürlichen Fettes (z.B. Pflanzenöle oder tierische Fette) und 35 Gewichtsteilen eines Creme- bzw. Salbengrundstoffs vermischt.
Das Fett dient dem Zweck, die Hautverträglichkeit der Creme zu erhöhen und das Einziehen der Wirkstoffe in die Haut noch zu fördern.

### Shaho-B (Creme/Salbe)

100 Gewichtsteile der Shaho-Creme A werden mit 25 bis 50 Gewichtsteilen Knoblauchsaft vermischt.
Die Shaho-Creme B zeigt eine besonders gute Wirkung bei der Behandlung von kreisrundem Haarausfall.

### Shaho-C (Creme/Salbe)

100 Gewichtsteile der Shaho-Creme A werden mit 25 Gewichtsteilen Knoblauchsaft und 30 Gewichtsteilen Apfelessig vermischt.
Die Shaho-Creme C zeigt eine besonders gute Wirkung bei der Behandlung von Schuppenflechte.

### Shaho E (Flüssigpräparat)

Der gemäß Beispiel 1 oder Beispiel 2 hergestellte eingedickte Sud wird mit destilliertem Wasser im Mischungsverhältnisse 1 Gewichtsteil Wasser auf 4 bis 10 Gewichtsteile Sud zu einem dünnflüssigen Präparat vermischt. Dazu kommen 2% (des Gesamtgewichts von Wasser und Sud) Vaseline oder Cremegrundfett und werden mit der Sud-Wasser-Mischung gut verschüttelt. Vor der Benutzung sollte bzw, muß dieses Flüssigpräparat nochmals gut geschüttelt werden.
Dünnflüssigere Präparate haben eine niedrigere Wirkstoffkonzentration, lassen sich aber leichter zwischen noch vorhandenen Haaren auf die Kopfhaut auftragen. Aus diesem Grund sind dünnflüssige Shaho-E-Präparate insbesondere für den Einsatz bei Patienten mit geringem Haarausfall und noch viel vorhandenem Haar oder bei Patienten mit dünnem Haar und den Beschwerden Kopfschuppen oder Kopfjucken vorgesehen.
Zähflüssigere Shaho-E-Präparate sind dagegen insbesondere für Patienten mit wenig oder keinen Haaren geeignet, da in diesen Fällen viel Wirkstoff erwünscht ist, und das Auftragen auf die Kopfhaut kein Problem darstellt.

### Shaho-F (Flüssigpräparat)

Der gemäß Beispiel 2 hergestellte, noch nicht eingedickte Sud wird mit destilliertem Wasser im Verhältnis 4:1 (4 Teile Sud + 1 Teil Wasser) verdünnt und in dieser Form als Flüssigpräparat Shaho-F eingesetzt. Diese Shaho-F ist einfach zu benutzen (weil relativ dünnflüssig), erreicht die Haarwurzeln schnell und zeigt bei täglicher Anwendung (8-stündige Einwirkdauer) bereits innerhalb von 7 Tagen erste positive Wirkungen.
Das Flüssigpräparat Shaho-F weist im Prinzip dieselben Vorteile wie das Flüssigpräparat Shaho-E auf.
Beide Präparate sind insbesondere für die Anwendung bei solchen Patienten vorgesehen, die nur leichten Haarausfall haben und nicht bereit sind, für die Behandlung ihr Haar abzurasieren.
Die leichte Anwendbarkeit von Shaho-E und Shaho-F erlaubt auch eine prophylaktische Anwendung.

### Beispiel 5: Probandentest mit den Creme-Präparaten Shaho-A, Shaho-B und Shaho-C

Im Probandentest wurden die gemäß Beispiel 4 hergestellten Creme-Präparate Shaho-A, Shaho-B und Shaho-C erfolgreich gegen Haarausfall, für die Vitalisierung vorhandener Haarwurzeln, zur Linderung von Hautjucken aufgrund von Allergien und anderen Ursachen und zur Wiederbelebung der Haarpigmentproduktion eingesetzt.
Je nach Schwere des Haarausfalls wurden die Probanden (Testpersonen) in die Grade 1 (leicht) bis 7 (sehr schwer) eingeteilt.
Die Mehrzahl der Probanden hatten sich die Haare für die Dauer der Behandlung abrasieren lassen. Lediglich eine Frau und vier Männer mit Haarausfall Grad 1, Grad 2 und Grad 3 hatten sich die Haare nicht abrasieren lassen.
Vor dem Auftragen des Präparats wurde die Kopfhaut 5 bis 10 Minuten massiert, danach der Kopf gewaschen und vollständig abgetrocknet, teilweise trocken geföhnt. Das Präparat wurde anschließend direkt auf die Kopfhaut aufgetragen und einmassiert (vorhandene Haare wurden zur Seite gekämmt). Je nach zu behandelnder Fläche wurden 1 bis 5 cm³ des Präparats aufgetragen, wobei die Menge 5 cm³ bei Behandlung des gesamten Kopfes aufgetragen wurde.
Während der Einwirkzeit erfolgte eine Wärmebehandlung, z.B. durch das Tragen einer warmen Kopfbedeckung oder durch einen Saunabesuch.
Die Einwirkzeit pro Behandlung betrug in der Regel zwischen 1 und 8 Stunden. 1 Stunde genügte, wenn als Wärmebehandlung ein Saunagang erfolgte. 8 Stunden (z.B. eine Nacht) war in den allermeisten Fällen völlig ausreichend. 72 Stunden war die maximale Einwirkzeit und wurde bei Frauen mit kräftigen langen Haaren durchgeführt. (Bei dieser Personengruppe werden die Wirkstoffe erfahrungsgemäß am langsamsten aufgenommen).
Nach dem Ende der Einwirkzeit (siehe unten) wurde das Präparat ausgewaschen.
Bei Einwirkzeiten bis zu 8 Stunden wurde jeden Tag behandelt; bei einer Einwirkzeit von 72 Stunden einmal pro Woche.

Die Ergebnisse dieser Tests sind in den Tabellen 1-7 dargestellt. Die Tabellen sind jeweils für einen Schweregrad des Haarausfalls zusammengestellt.
In diesen (und den weiteren Tabellen) bedeutet:
- "Personen-Nr." die Aktennummer der Versuchsdokumentation;
- "A", "B" oder "C" in der Spalte "Typ" das Präparat Shaho-A bzw. Shaho-B bzw. Shaho-C;
- "+" in der Spalte "Ernährung" bedeutet, daß zusätzlich zur Behandlung mit dem Shaho-Präparat ein spezielles Ernährungsprogramm durchlaufen wurde, "-" in dieser Spalte bedeutet, daß kein solches Programm durchlaufen wurde;
- "+" in der Spalte "Sport" bedeutet, daß zusätzlich zur Behandlung mit dem Shaho-Präparat ein spezielles Sport- bzw. Bewegungsprogramm durchlaufen wurde, "-" in dieser Spalte bedeutet, daß kein solches Programm durchlaufen wurde; "Rasur" steht für das Abrasieren der Haare für die Dauer der Behandlung;
- in der Spalte "Rasur" bzw. "Rasieren" bedeutet "+" das völlige Glattrasieren der Kopfhaut für Behandlungsdauer, "-" bedeutet, daß keine Rasur stattfand;
- "Menge in cm" die für die Dauer Behandlung an den Probanden abgegebenen Gesamtmenge des Präparats sowie Anzahl und Größe der Einzelportionen; beispielsweise stehen "45+45" in dieser Spalte für "2 Portionen ä 45 cm^{3"}.
- Die Behandlungsdauer ist z.T. in einzelne Blöcke von mehreren aufeinanderfolgenden Tagen unterteilt. Diese Blöcke können direkt aufeinanderfolgen oder Tage bis mehrere Monate auseinander liegen. Über solche Pausen wird in der Tabelle keine Angabe gemacht. So steht beispielsweise "30+30" in der betreffenden Spalte "Behandlungsdauer in Tagen" für 2 Blöcke ä 30 Tage mit unbekannt langer Behandlungspause. "+" in der Spalte "Wirkung" bedeutet "Wirkung nachweisbar vorhanden", "-" in dieser Spalte bedeutet "keine Wirkung erkennbar".

Generell gilt, daß beste Wirkung erzielt werden, wenn vorhandene Haare vollständig abrasiert werden und das Präparat unter zusätzlicher Wärme einwirken kann. Eine warme Mütze, warme Sonne oder ein Saunabesuch verstärken die Wirkung erheblich. Die Einwirkzeit liegt dabei in der Größenordnung von Stunden bis zu drei Tagen. Je länger das Präparat auf die Haut einwirken kann, desto besser die Wirkung. Das Präparat einzumassieren verbessert die Durchblutung der Haut und erhöht ebenfalls die Wirkung.

Die Erfahrung zeigt; Männer benötigen eine kürzere Einwirkzeit als Frauen, kräftiges Haar (z.B. bei einer Behandlung gegen graues Haar) bedingt eine längere Einwirkzeit als die Behandlung kahler Stellen.

Die Behandlungshäufigkeit und die gesamte Behandlungsdauer können individuell an den jeweiligen Patienten angepaßt werden.

### Beispiel 6: Probandentests mit den Flüssigpäparaten Shaho-E und Shaho-F

Im Probandentest wurde das gemäß Beispiel 4 hergestellte Flüssigpräparat Shaho E erfolgreich gegen Haarausfall, für die Vitalisierung vorhandener Haarwurzeln , zur Linderung von Hautjucken aufgrund von Allergien und anderen Ursachen und zur Wiederbelebung der Haarpigmentproduktion eingesetzt.
Die Testpersonen (eine Frau und vier Männer mit Haarausfall Grad 1 , Grad 2 und Grad 3) hatten die Haare nicht abrasiert. 1 mal am Tag wurde die Kopfhaut massiert, Shaho-E aufgetragen und ca. 8 Stunden einwirken lassen. haben sie benutzt.
Das Ergebnis dieses Tests ist in Tabelle 8 dargestellt.

Im direkten Vergleich hierzu wurde das gemäß Beispiel 4 hergestellte Flüssigpräparat Shaho F im Probandentest eingesetzt. Der Test wurden unter den gleichen Bedingungen wie für das Flüssigpräparat Shaho E beschrieben durchgeführt.

Das Ergebnis dieses Tests ist in Tabelle 9 dargestellt.

### Beispiel 7: Probandentest zur Anwendung von Shaho-Creme-Präparaten speziell bei Frauen mit Haarausfall

Insbesondere bei Frauen (selbstverständlich aber auch bei Männern), die ihr noch vorhandenes Haar nicht abrasieren wollen, wird zur Bekämpfung von Haarproblemen folgende Behandlungsweise vorgeschlagen:
Pro Behandlung werden 15-20 cm³ eines gemäß Beispiel 4 hergestellten Shaho-Creme-Präparates (A, B oder C) auf die Kopfhaut aufgetragen, einmassiert und zwischen 24-72 Stunden einwirken gelassen. Während der Einwirkungszeit sollte eine Wärmebehandlung erfolgen, z.B. durch das Tragen einer warmen Kopfbedeckung.
Bei einer Einwirkungszeit von 72 Stunden wird die Behandlung im Abstand von jeweils 10 Tagen noch zweimal wiederholt (d.h. 3 mal in einem Monat).

Im Probandentest wurde insgesamt 13 Frauen unterschiedlichen Alters und mit unterschiedlicher Haarausfallursache dementsprechend behandelt. Der genaue Behandlungsplan und die Ergebnisse sind in Tabelle 10 dargestellt. Aus dieser Tabelle ist ersichtlich, daß die Behandlung bei allen Testpersonen sehr erfolgreich verlief: in den meisten Fällen wurde der Haarausfall vollständig gestoppt und es kam zu verstärktem Haarneuwachstum.

### Beispiel 8: Probandentest zur Behandlung von Schuppenflechte (Psoriasis) :

Zwei Testpersonen (Nr. 32 und Nr. 44), die an Schuppenflechte auf der Kopfhaut und zusätzlich an androgenem Haarausfall litten, wurden mit dem der gemäß Beispiel 4 hergestellten Shaho-Creme-Präparat Shaho C über einen Zeitraum von mehreren Tagen behandelt. Der genaue Behandlungsplan und die Ergebnisse sind in Tabelle 1 und 5 dargestellt. Auch in diesen Krankheitsfällen konnte durch eine Behandlung mit dem Shaho-Präparat eine deutliche Besserung des Befindlichkeitszustandes erreicht werden: Sowohl bei der Person Nr. 32 mit Haarausfall Grad 5 (siehe Tabelle 5) als auch bei der Person Nr. 44 mit Haarausfall Grad 1 (siehe Tabelle 1) wurde eine deutliche Besserung der Psoriasis-Symptome erreicht.

### Beispiel 9: Probandentest zur Behandlung von kreisrundem Haarausfall (Alopecia)

Im Probandentest wurden 12 Personen behandelt, die unterschiedlich schwer von Alopecia betroffen waren. Die Probanden wurden mit dem der gemäß Beispiel 4 hergestellten Shaho-Creme-Präparate Shaho B über einen Zeitraum von 7 bis 30 Tagen behandelt. Der genaue Behandlungsplan und die Ergebnisse sind in Tabelle 11 dargestellt. Aus dieser Tabelle 11 ist ersichtlich, daß die Behandlung bei allen Testpersonen sehr erfolgreich verlief:

Bei 8 Testpersonen waren die Symptome von *Alopecia areata* nachweisbar. Davon wiesen 2 Testpersonen jeweils einen Kreis mit 2-3 cm Durchmesser auf, die Zeit der Infektion betrug in diesen beiden Fällen weniger als einen Monat. Nach der Behandlung waren Haarausfall und Verbreitung des Kreises gestoppt und neue Haaren zu 100% nachgewachsen. Eine Testperson litt seit über 6 Monaten an 5 Kreisen mit je einem Durchmesser von etwa 1 cm. Infolge der Behandlung kamen Haarausfall und Verbreitung des Kreises zu 100% zum Stillstand und erstes weiches Haar wuchs nach. Zwei Jahre später wurden dieser Testperson im Bereich der ursprünglichen Alopecia-Kreise Hydrocortison gespritzt und dadurch Haarneuwachstum von 100% erreicht. Bei einer anderen Testperson, die unter mehreren großen kahlen Alopecia-Kreise litt und seit vielen Jahren von der Krankheit betroffen war, wurde die Ausdehnung der Kreise zu 100% gestoppt und neues Haar wuchs nach. Bei einer weiteren Testperson, die seit 2 Jahren unter der Krankheit litt, wies der Alopecia-Kreis bei Behandlungsbeginn einen Durchmesser von ungefähr 15 Zentimeter auf. Infolge der Behandlung mit dem Shaho-Präparat wurde die weitere Ausdehnung des Kreises zu 100% gestoppt, die Poren öffneten sich vollständig, und 3 Jahre nach der Behandlung war der betroffene Bereich deutlich kleiner geworden, sein Durchmesser betrug nur noch etwa 8 cm und erstes weiches Haar war bereits im Randbereich nachgewachsen. Eine der Testpersonen war betroffen von kleinen Kreisen mit nassen Wunden, Jucken und Pilzbefall auf der Kopfhaut. Nach einem Monat Behandlung waren der Haarverlust vollständig gestoppt, das Jucken hatte aufgehört, die Wunden waren verschwunden, und das Haar wuchs in diesen Kreisen wieder zu 100% nach. Eine Testperson machte keine Angaben zu den Wirkungen der Behandlung, eine weitere Testperson gab mündlich an, daß ihr die Behandlung geholfen hatte.

Bei einer Testperson waren die Symptome von *Alopecia areata totalis* nachweisbar. Diese Person litt unter vollständigem Haarausfall, ihre Kopfhaut war relativ unbeweglich, die Hautporen waren vollständig-geschlossen, an den betroffenen Stellen waren Wunden mit Juckreiz vorhanden, und die Haut war sehr fettig. Die Behandlung mit dem Shaho-Präparat B zeigte folgende Wirkungen: Die Poren öffneten sich vollständig, die normale Dicke der Kopfhaut wurde wieder hergestellt und die Beweglichkeit der Kopfhaut erhöhte sich, die Wunden verheilten vollständig, der Juckreiz verschwand, erstes weiches Haar wuchs nach und fettige Anlagerungen waren deutlich weniger vorhanden.

**Tabelle 1: Probandentest mit Haarausfall 1. Grades**

| **Personen** | | **Shaho Creme** | | | **Gebrauchsanweisung** | | | **Stop von** Haarausfall | | **Wirkung** | | |
|---|---|---|---|---|---|---|---|---|---|---|---|---|
| Nr. | Geb. datum | Typ | Menge in cm³ | Behandlungsdauer | Sport | Ernährung | Rasieren | Prozent | Nach Anzahl Tagen | Haarwachstum | Vitalisierung vorhandener Haarwurzeln | Hautkrankheiten |
| 20 | 1972 | A | 45 | 30 | + | + | - | 90 | 7 | + | + | + |
| 28 | - | A | 30 | 14 | - | - | - | 100 | 15 | + | + | + |
| 41 | - | A | 45 | 30 | - | - | + | 100 | 15 | + | + | + |
| 44 | 1972 | A | 45 | 30 | - | - | - | 90 | 14 | + | + | + |
| 50 | 1972 | A | 45 | 30 | - | - | - | 75 | 14 | + | + | + |
| 53 | 1968 | A | 45 | - | - | - | - | - | - | + | + | + |
| 54 | - | A | 45 | 30 | - | - | - | - | - | + | + | + |
| 57 | 1950 | A | 90 | 14 | - | - | - | 95 | - | + | + | + |
| 61 | - | A | 45 | - | - | - | - | 95 | - | + | + | + |
| 66 | - | A | 12 | - | - | - | - | - | - | + | + | + |
| 71 | - | A | 30 | - | - | - | - | - | - | + | + | + |
| 74 | - | A | 12 | - | - | - | - | - | - | + | + | + |
| 101 | 1978 | A | 45 | 30 | - | + | - | 95 | - | + | + | + |
| 67 | 1969 | A | 45 | 25 | - | - | - | 90 | 30 | - | - | - |
| 72 | 1975 | A | 12 | 10 | - | - | - | 100 | - | + | + | + |
| 89 | 1981 | A | 20 | 7 | - | - | + | 100 | 10 | + | + | + |
| 90 | 1978 | A | 45 | 30 | - | - | + | 95 | - | + | + | + |
| 91 | 1979 | A | 12 | 7 | - | - | - | 60 | 14 | + | + | + |
| 92 | 1970 | A | 12 | 7 | - | - | + | 100 | 15 | + | + | + |
| 93 | 1973 | A | 30 | 25 | - | - | + | 100 | 30 | + | + | + |
| 94 | 1973 | A | 12 | 10 | - | - | - | 95 | 30 | + | + | + |
| 95 | 1977 | A | 12 | 8 | - | - | + | 90 | - | + | + | + |

**Tabelle 2 : Probandentest mit Haarausfall 2. Grades**

| **Personen** | | **Shaho-Präpaparat** | | | **Gebrauchsanweisung** | | | **Stopp von Haarausfall** | | **Wirkungen** | | |
|---|---|---|---|---|---|---|---|---|---|---|---|---|
| Nr. | Geb. Datum | Typ | Menge in cm³ | Behandlungsdauer | Sport | Ernährung | Rasur | Prozent | Nach Anzahl Tagen | Haarwachstum | Vitalisierung vorhandener Haarwurzeln | Hautkrankheiten |
| 2 | 1948 | A | 45+45+45 | 20+15+20 | - | - | + | 100 | 7 | + | + | + |
| 3 | 1964 | A | 90+45 | 60+30+6 | - | + | - | 100 | 30 | + | + | + |
| 14 | 1970 | A | 45 | 25+25 | + | + | - | 75 | 15 | + | + | + |
| 15 | 1975 | A(1/2) | 45 | 30 | + | + | - | 20 | 14 | + | + | + |
| 16 | 1970 | A | 45 | 30 | - | - | - | 100 | 14 | + | + | + |
| 25 | 1974 | A | 45 | 40 | + | + | + | 95 | - | + | + | + |
| 26 | 1962 | A | 45 | - | - | + | - | - | - | + | + | + |
| 31 | 1967 | A | 45 | 30 | + | + | + | 100 | - | + | + | + |
| 33 | 1965 | A | 45 | 23 | + | + | - | 90 | | + | + | + |
| 35 | - | A | 45 | 30 | + | - | - | 100 | | | | |
| 43 | 1978 | A | 45 | 31 | + | - | - | 100 | - | + | + | + |
| 46 | 1974 | A | 45 | - | - | - | - | - | - | + | + | + |
| 56 | - | A | 45 | - | - | - | - | - | - | + | + | + |
| 63 | 1980 | A | 20 | 10 | - | + | - | 100 | - | + | + | + |
| 68 | 1942 | A | 45+45 | - | - | - | - | - | - | + | + | + |
| 75 | - | A | 12 | - | - | - | - | | - | + | + | + |
| 103 | 1975 | A | 50 | 30 | - | - | - | | | + | + | + |
| 88 | 1965 | A | 20 | 12 | - | - | + | 100 | 15 | + | + | + |
| 82 | 1956 | A | 12 | 10 | - | - | + | 100 | 15 | + | + | + |
| 83 | 1966 | A | 14 | 14 | - | - | + | 90 | 60 | + | + | + |
| 85 | 1974 | A | 12 | 12 | - | - | + | 85 | 15 | + | + | + |
| 96 | 1972 | A | 20 | 12 | - | - | + | 80 | 15 | + | + | + |
| 97 | 1975 | A | 50 | 30 | - | - | + | 100 | 15 | + | + | + |

**Tabelle 3 : Probandentest mit Haarausfall 3. Grades**

| **Personen** | | **Shaho Creme** | | | **Gebrauchsanweisung** | | | **Stop von Haarausfall** | | **Wirkung** | | |
|---|---|---|---|---|---|---|---|---|---|---|---|---|
| Nr. | Geb. datum | Typ | Menge in cm³ | Behandlungsdauer in Tagen | Sport | Ernährung | Rasur | Prozent | Nach Anzahl Tagen | Haarwachstum | Vitalisierung vorhandener Haarwurzeln | Hautkrankheiten |
| 9 | 1973 | A | 45 | 32 | + | + | - | 0 | - | + | - | - |
| 12 | - | A | 45 | - | - | - | - | + | - | + | + | + |
| 13 | 1973 | A | 45+45+45 | 45+8+3+2 | + | + | - | 100 | 30 | + | + | |
| 17 | 1965 | A | 45+45 | 30+20 | + | + | - | 85 | 21 | + | + | + |
| 37 | - | A | 12 | 7 | - | - | - | 75 | 8 | + | + | + |
| 42 | 1960 | A | 45 | 30 | + | + | - | 100 | 1 | + | + | + |
| 47 | - | A | 30 | - | - | - | - | - | - | - | - | - |
| 48 | - | A | 45 | 30 | - | - | | 100 | + | + | + | + |
| 49 | - | A | 45 | - | - | - | - | - | - | - | - | - |
| 60 | - | C | 60 | - | - | - | | 100 | - | + | + | + |
| 70 | - | A | 12 | 5 | - | - | + | | | | | - |
| 78 | 1980 | A | 25 | 15 | - | - | + | 100 | 5 | + | + | + |
| 79 | 1963 | A | 45 | 7 | - | - | + | 95 | 15 | + | + | + |
| 86 | 1974 | A | 15 | 30 | - | - | + | 98 | 7 | + | + | + |
| 98 | 1972 | A | 25 | 14 | - | - | + | 95 | 14+ | + | + | + |

**Tabelle 4 : Probandentest mit Haarausfall 4. Grades**

| **Personen** | | **Shaho Creme** | | | **Gebrauchsanweisung** | | | **Stop von Haarausfall** | | **Wirkung** | | |
|---|---|---|---|---|---|---|---|---|---|---|---|---|
| Nr. | Geb. datum | Typ | Menge in cm³ | Behandlungsdauer | Sport | Ernährung | Rasieren | Prozent | Nach Anzahl Tagen | Haarwachstum | Vitalisierung vorhandener Haarwrzeln | Hautkrankheiten |
| 18 | 1971 | A | 45+45 | 30 | + | + | - | 70 | 20 | + | + | + |
| 19 | 63 | A | 45+45+45+12 | 30+30+30+7 | + | + | + | 95 | 15 | + | + | + |
| 29 | 1971 | A | 45+45+45+45 | 30+30+30+30 | + | - | + | 90 | 17 | + | + | + |
| 39 | 1971 | A | 45 | 30 | - | - | - | 95 | 15 | + | + | + |
| 58 | 1951 | A | 45 | 7 | - | - | - | 60 | 60 | + | + | + |
| 62 | - | A | 12 | 2+2 | | | - | | | | | |
| 73 | | A | 45 | | - | - | | | | + | - | - |
| 81 | 1965 | A | 12 | 7 | - | - | + | 80 | 10 | + | + | + |
| 87 | 1968 | A | 12 | 15 | - | - | + | | 30 | + | + | + |

**Tabelle 5: Probandentest mit Haarausfall 5. Grades**

| **Personen** | | **Shaho Creme** | | | **Gebrauchsanweisung** | | | **Stop von Haarausfall** | | **Wirkung** | | |
|---|---|---|---|---|---|---|---|---|---|---|---|---|
| Nr. | Geb. datum | Typ | Menge in cm³ | Behandlungsdauer | Sport | Ernährung | Rasieren | Prozent | Nach Anzahl Tagen | HaarWachstum | Vitalisierung vorhandener Haarwurzeln | Hautkrankheiten |
| 5 | 1968 | A | 45+45+45+45 | 20+25+40+25 | - | - | - | 100 | + | + | + | + |
| 7 | 57 | A | 45 | 30 | + | + | - | 100 | + | + | + | + |
| 11 | 73 | A | 45+45+45 | 20+2+30+3 | + | - | - | 90 | + | + | + | + |
| 22 | 1965 | A | 45+45 | 30+27+30+30 | + | + | - | 100 | | | - | |
| 27 | 1956 | A | | | | | | | + | + | + | + |
| 32 | 1973 | A | 45+45 | 30+30 | + | + | + | 100 | + | + | + | + |
| 38 | 1972 | A | 45+45 | 30+30 | + | -- | - | 90 | + | + | + | + |
| 40 | 1965 | A | 45+45 | 25 | + | + | -- | 100 | | | | |
| 65 | | A | - | | | | | | | | | |
| 69 | | A | | 12 | - | | | | | | | |
| 73 | | A | 12 | - | | | | | | | | |
| 77 | | A | 45 | - | | | | | | | | |
| 99 | 1971 | A | 45 | 15 | - | - | + | 100 | 15 | + | + | + |

**Tabelle 6 : Probandentest mit Haarausfall 6. Grades**

| **Personen** | | **Shaho Creme** | | | **Gebrauchesanweisung** | | | **Stop von Haarausfall** | | **Wirkung** | | |
|---|---|---|---|---|---|---|---|---|---|---|---|---|
| Nr. | Geb. datum | Typ | Menge in cm³ | Behandlungsdauer | Sport | Ernährung | Rasieren | Prozent | Nach Anzahl Tagen | HaarWachstum | Vitalisierung vorhandener Haarwurzeln | Hautkrankheiten |
| 30 | | A | 45+45+45 | | | | | 100 | | | | |
| 34 | | A | 45 | 30 | | | | - | | | | |
| 36 | 52 | A | 45+45+45 | 2+30+20 | | | | - | | | | |
| 51 | | D | 45 | 7 | | | | - | | + | - | |
| 80 | 1971 | A | 12 | 9 | - | - | + | 90 | + | + | + | + |

**Tabelle 7 : Probandentest mit Haarausfall 7. Grades**

| **Personen** | | **Shaho Creme** | | | **Gebrauchesanweisung** | | | **Stop von Haarausfall** | | **Vorteilen** | | |
|---|---|---|---|---|---|---|---|---|---|---|---|---|
| Nr. | Geb. datum | Typ | Menge in cm³ | Behandlungsdauer | Sport | Ernährung | Rasieren | Prozent | Nach einige Tage | Haarwachstum | Vitalisierung vorhandener Haarwurzeln | Hautkrankheiten |
| 1 | 1956 | A | 45 | 9 | - | - | - | 90 | 30 | | - | |
| 4 | 1954 | A | 45+45 | 60 | - | + | - | | | | | |
| 6 | 1959 | A+B | 45+45 | 30+30 | - | + | - | | | | | |
| 8 | 1956 | A | 45 | 15 | - | + | - | 30 | 7 | | | |
| 10 | 1946 | A | 45+45 | 60+20 | + | - | - | 100 | 7 | | | |
| 21 | 1962 | A | 45+45 | 30 | - | - | - | | | | | |
| 23 | 1947 | A | 45+45 | 30+30 | - | + | - | 25 | | | | |
| 24 | 1957 | A | 45 | 30 | - | - | - | 100 | 14 | | | |
| 52 | | D | 45 | - | - | - | - | | | - | | |
| 78 | 1936 | D | 45 | - | - | - | - | | | | | |
| 59 | 1954 | A | 10 | 5 | - | - | - | + | + | + | + | + |
| 84 | 1953 | A | 45 | 30 | - | - | - | + | - | + | + | + |
| | | | | | | | | | | | | |

**Tabelle 8: Behandlungsergebnisse des Probandentests mit Shaho-E:**

| **Nr.** | **Stop von Haarausfall** | **Grad des Haarausfalls** | **Vitalisierung vorhandener Haarwurzeln** | **Haarwachstum** | **Behandlungsdauer in Tagen** | **Andere+ Wirkungen** | **Geschlecht + Alter** |
|---|---|---|---|---|---|---|---|
| 1 | 90% | 2 | + | + | 15 | Jucken + Schuppen geheilt | M 24 |
| 2 | 85% | 1 | + | - | 7 | | M 35 |
| 3 | 100% | 3 | + | - | 20 | | M 38 |
| 4 | 95% | 2 | + | + | 20 | Jucken + Pickel geheilt | M 28 |

**Tabelle 9: Behandlungsergebnisse des Probandentests mit Shaho-E:**

| **Nr.** | **Stop von Haarausfall** | **Grad des Haarausfalls** | **Vitalisierung vorhandener Haarwurzeln** | **Haarwachstum** | **Behandlungs-Dauer in Tagen** | **Andere Wirkungen** | **Geschlecht + Alter** |
|---|---|---|---|---|---|---|---|
| 1 | 100% | 2 | + | + | 15 | | M 28 |
| 2 | 90% | 2 | + | - | 7 | Jucken + schuppen geheilt | M 26 |
| 3 | 95% | 1 | + | - | 7 | | M 38 |
| 4 | 100% | --- | + | + | 20 | Jucken + Pickel geheilt | M28 |
| 5 | 100% | --- | + | ++ | 15 | --- | F 35 |
| 6 | 100% | --- | + | ++ | 15 | | F33 |

**Tabelle 10: Behandlungsergebnisse des Probandentests speziell an Frauen:**

| **Personen** | | **Shaho Creme** | | **Wirkung** | | |
|---|---|---|---|---|---|---|
| Nr. | Alter | Typ | Menge in cm³ | Stop von Haarausfall | Haarwachstum | Vitalisierung vorhandener Haarwurzeln |
| 1 | 50 | A | 20 | 100% | + | + |
| 2 | 45 | A | 20 | 95% | + | + |
| 3 | 35 | A | 20 | 85% | + | + |
| 4 | 27 | A | 20 | 100% | + | + |
| 5 | 17 | A | 20 | 100% | + | + |
| 6 | 25 | A | 20 | 100% | + | + |
| 7 | 35 | A | 20 | 100% | + | + |
| 8 | 32 | A | 20 | 95% | + | + |
| 9 | 38 | A | 20 | 100% | + | + |
| 10 | 18 | A | 20 | 100% | + | + |
| 11 | 19 | A | 20 | 100% | + | + |
| 12 | 75 | A | 20 | 100% | - | - |
| 13 | 40 | A | 20 | 85% | + | + |

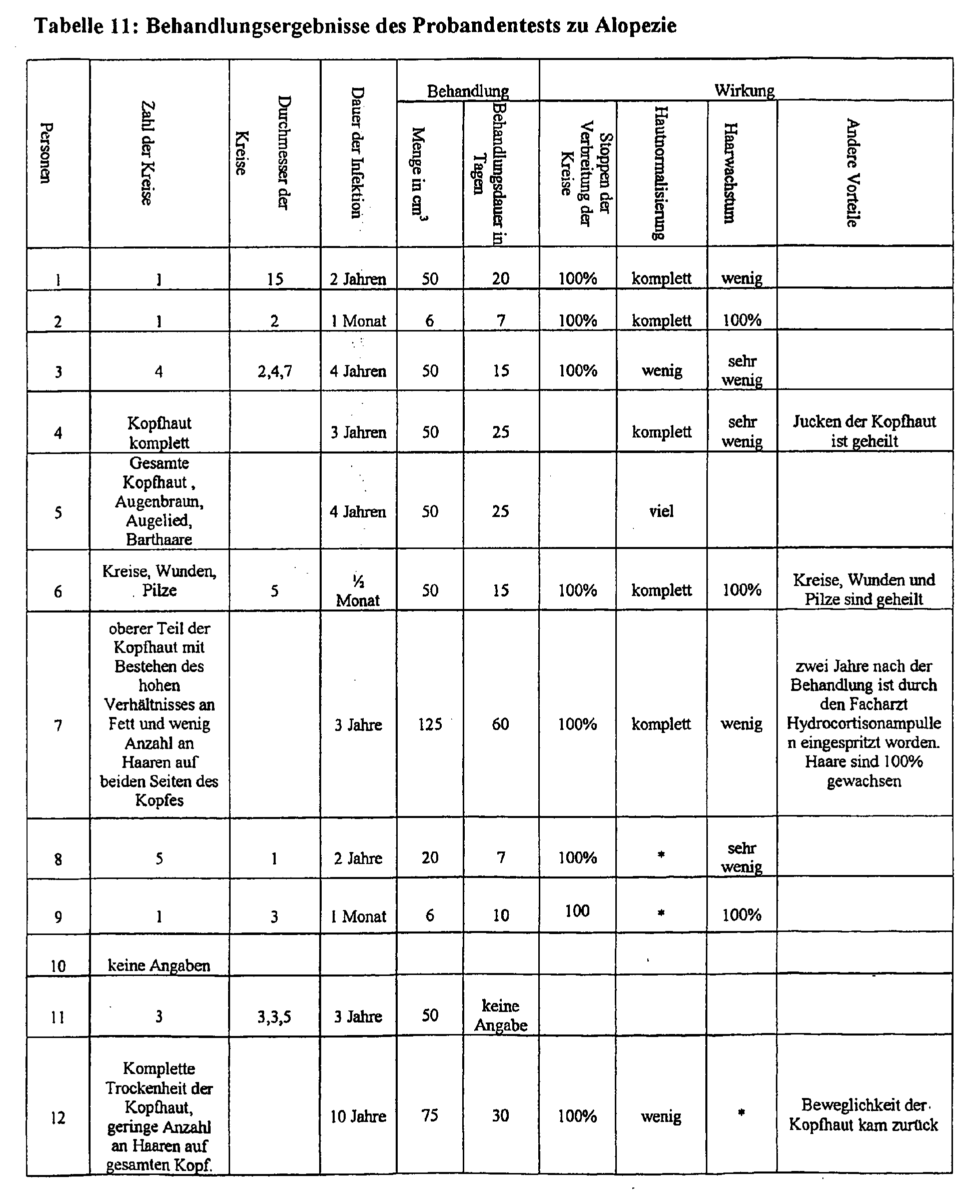

## Patentansprüche

1. Wirkstoffmischung, **gekennzeichnet dadurch, dass** sie aus einer Pflanze der Art *Convolvulus euphraticus Bornm.* gewonnen wird, indem aus der Pflanze entweder (i) ein wässriger Sud durch Kochen in Wasser oder (ii) ein alkoholischer Auszug durch Einlegen in Alkohol oder (iii) eine Paste durch Verarbeitung der fein zerriebenen Pflanze mit hautverträglichen mehr oder minder viskosen Flüssigkeiten gewonnen wird, wobei der Sud bzw. der Auszug bzw. die Paste die Wirkstoffmischung enthält.

2. Wirkstoffmischung nach Anspruch 1 zur Anwendung in einem therapeutischen und/oder kosmetischen Verfahren.

3. Wirkstoffmischung nach Anspruch 1 als Mittel gegen Haarausfall, als Mittel für die Heilung von Schuppenflechte, als Mittel für die Heilung von Infektionskrankheiten der Haut, als Mittel für die Linderung von Hautjucken oder als Mittel für die Linderung von Neurodermitis.

4. Verwendung der Wirkstoffmischung gemäß Anspruch 1 zur Herstellung eines pharmazeutischen Mittels gegen Haarausfall, für die Heilung von Schuppenflechte, für die Heilung von Infektionskrakheiten der Haut, für die Linderung von Hautjucken oder für die Linderung von Neurodermitis.

5. Nicht-therapeutischeVerwendung der Wirkstoffmischung gemäß Anspruch 1 als Mittel gegen Haarausfall oder als Mittel welches die Hautpigmentproduktion anregt und damit die Zunahme grauer Haare stoppt und ergraute Haare in ihrer ursprünglichen Farbe weiterwachsen läßt.

## Claims

1. Mixture of active agents, **characterised by** the fact that it is obtained by producing from a plant of the species *Convolvulus euphraticus Bornm.* either (i) an acqueous decoction by cooking it in water or (ii) an alcoholic extract by placing it in alcohol or (iii) a paste by processing the finely ground plant with skin-friendly more or less viscous liquids, whereby the decoction or the extract or the paste contains the mixture of active agents.

2. Mixture of active agents according to Claim 1 for use in a therapeutic and/or cosmetic procedure.

3. Mixture of active agents according to Claim 1 as a remedy for hair loss, as a remedy for curing psoriasis, as a remedy for curing infectious skin diseases, as a remedy for relieving itchy skin or as a remedy for relieving neurodermatitis.

4. Use of the mixture of active agents pursuant to Claim 1 for producing a pharmaceutical remedy for hair loss, for curing psoriasis, for curing infectious skin diseases, for relieving itchy skin or for relieving neurodermatitis.

5. Non-therapeutic use of the mixture of active agents pursuant to Claim 1 as a remedy for hair loss or as a remedy that stimulates the production of skin pigments and thus stops the increase in grey hairs and allows greying hairs to continue to grow in their original colour.

## Revendications

1. Mélange de substances actives **caractérisé par** sa composition à base d'une plante du genre *Convolvulus euphraticus Bornm.,* soit (i) sous forme de décoction en l'infusant dans de l'eau bouillante, soit (ii) sous forme d'extrait alcoolique en la mettant dans de l'alcool, soit (iii) sous forme de pâte en mélangeant la plante finement broyée avec des liquides plus ou moins visqueux et doux pour la peau, sachant que la décoction ou l'extrait ou la pâte contient le mélange de substances actives.

2. Mélange de substances actives selon la revendication 1 pour une utilisation dans le cadre d'un procédé thérapeutique et /ou cosmétique.

3. Mélange de substances actives selon la revendication 1 en tant que remède contre la chute des cheveux, en tant que remède pour la guérison du psoriasis, en tant que remède pour la guérison de maladies infectieuses de la peau, en tant que remède pour le soulagement de prurits ou bien en tant que remède pour le soulagement de névrodermite.

4. Utilisation du mélange de substances actives selon la revendication 1 afin de préparer un remède pharmaceutique contre la chute des cheveux, pour la guérison du psoriasis, pour la guérison de maladies infectieuses de la peau, pour le soulagement de prurits ou pour le soulagement de névrodermite.

5. Utilisation non thérapeutique du mélange de substances actives selon la revendication 1 en tant que remède contre la chute des cheveux ou bien en tant que remède qui stimule la production de pigments de la peau et arrête ainsi l'augmentation de cheveux gris en les faisant repousser dans leur couleur d'origine.
